# EUROPEAN PATENT SPECIFICATION

(11) **EP 2 895 108 B1**
(45) Date of publication and mention of the grant of the patent: **31.05.2017**
(21) Application number: 13753766.8
(22) Date of filing: 19.08.2013
(51) Int. Cl.: A61F 2/01, A61B 17/12

(54) **FIXATION ANCHOR DESIGN FOR AN OCCLUSION DEVICE**
ENTWURF EINES FIXIERANKERS FÜR EINE OKKLUSIONSVORRICHTUNG
ANCRE DE FIXATION POUR DISPOSITIF D'OCCLUSION

(30) Priority: 12.09.2012 US 201261700221 P
(43) Date of publication of application: 22.07.2015
(73) Proprietor: Boston Scientific Scimed, Inc., Maple Grove, MN 55311 (US)
(72) Inventor: TISCHLER, Brian Joseph, Shoreview, Minnesota 55126 (US); PEIFFER, Dennis A., Brooklyn Park, Minnesota 55443 (US)
(74) Representative: Vossius & Partner Patentanwälte Rechtsanwälte mbB
(86) International application number: PCT/US2013/055572
(87) International publication number: WO 2014/042825

(56) References cited:
- EP-A1- 0 430 848
- WO-A2-2013/022567
- US-A1- 2008 300 620
- US-A1- 2011 054 515
- US-A1- 2012 245 619
- US-A1- 2013 035 713
- US-B1- 7 803 171

## Description

### BACKGROUND OF THE INVENTION

This invention relates to fixation anchors that may be incorporated into the structure of an occlusion device or vascular filter.

Medical devices implantable within the vasculature are often used to mitigate against blood clots, deep venous thrombosis, and/or pulmonary emboli when anticoagulation therapy is contraindicated. For example, a pregnant woman afflicted with atrial fibrillation may be implanted with a left atrial appendage occlusion device rather than prescribed anticoagulants. Once in place, the device provides scaffolding over which a tissue plug isolating the left atrial appendage may be formed. As another example, an elderly patient having just undergone a major surgery may be fitted with an inferior vena cava filter to mitigate against pulmonary emboli or deep venous thrombosis following surgery rather than prescribed anticoagulants. Once in place, the structure of the filter traps potential harmful blood clots that may occur as the patient recovers from surgery.

Both types of devices are generally implantable via endoscopic procedures utilizing catheter delivery systems. In general, a catheter carrying the device in a constrained state is advanced through the vasculature to the location of device deployment.

The device is then expanded and anchored in place by various barbs engaging the surrounding tissue.

US 7 803 171 B1 relates to an intravascular filter for minimally invasive deployment into, and extraction from, a blood vessel. The filter comprises an inferior vena cava filter used to prevent migration of clots into the pulmonary artery.

US 2011/0054515 A1 relates to an implantable medical device for insertion in the left atrial appendage which includes a cap coupled to a frame. The cap constrains movement of the legs of the frame during collapse and expansion of the device.

US 2008/0300620 A1 relates to an embolic filter that includes a hub and at least one elongated member extending from the hub. The at least one elongated member includes a core and a jacket circumscribing the core. A ratio of a core diameter to a jacket diameter is at least 0.60.

EP 0 430 848 A1 relates to a collapsible filter for introduction into a blood vessel of a patient and of the kind comprising a number of legs diverging from an apical hub and each having a reversely turned hook at its distal end with respect to said hub. Each leg comprises a central element, bent into a smooth quasi-halfsinusoidal form, and two substantially symmetrical curved side elements extending on either side of the central element. The filter may as a whole may be unfolded from a collapsed insertion condition in which the central and side elements of all legs forms a narrow bundle for arrangement in a catheter like insertion instrument into a tulip like filter configuration with the side elements interposed between the central elements of the legs. Without limiting the scope of the invention a brief summary of some of the claimed embodiments of the invention is set forth below. Additional details of the summarized embodiments of the invention and/or additional embodiments of the invention may be found in the Detailed Description of the Invention below.

### BRIEF SUMMARY OF THE INVENTION

The present invention is defined by the features of the claims.

An implantable medical device having barbs that reduce in height and change angle as the implantable device transitions from an expanded state to a constrained state may lessen the risk of injury associated with the removal and/or repositioning of the deployed device. Such a medical device, implantable within and retrievable from the vasculature of a patient, may comprise a main body formed from a plurality of ribs extending radially outwards from a nexus that are capable of transitioning from an expanded state to a collapsed state. The plurality of ribs defines an interior of the main of the body. Within the main body may be positioned a plurality of fixation struts. When the main body is in the expanded state, the fixation struts may extend through and across at least a portion of the interior. An end of each fixation strut may be secured to the main body directly or indirectly via an intermediary structure. A barb end of each fixation strut may exit the main body at a point of egress and project radially outwards from the main body. Each of the fixation struts may be secured to the main body, such that the distance between the point of attachment of the secured end of a fixation strut, and the respective point of egress from the main body of its free barb forming end is greater in the constrained state than in the expanded state, thereby reducing the height the barb end projects from the main body.

During implantation, the main body transitions from a constrained state to an expanded state. When in the expanded state, the free barb ends of each fixation strut extend radially outward past the outer diameter of the main body. Extending radially outward, the free barb ends of the fixation struts may collectively anchor the implantable medical device in place by engaging surrounding tissue.

While the inclusion of barbs on an implantable medical device may be beneficial in reducing migration, they may also be burdensome when repositioning and/or removing the implantable medical device. For example, if an implantable medical device is deployed at an inappropriate and/or less than ideal location, it may be desirable to reposition the device to a more ideal location. Moving the implantable medical device in the expanded state, however, may cause damage to the surrounding tissue as a consequence of barbs engaging the tissue.

Additionally, engagement of the surrounding tissue by barbs may cause complications when an implantable medical device is removed. For example, when an implantable medical device has served its function, it may be desirable to remove it as to prevent complications arising from its continued presence within the body. Removal of an implantable medical device may be done by transitioning an implantable medical device to the constrained state and recapturing it within a catheter. The transition to the constrained state and/or movement of an implantable medical device into a catheter may cause the barbs to traumatically disengage from the surrounding tissue thereby causing injury and/or other complications. The barbs may also damage the catheter during recapture, which may cause a release of debris from the catheter and/or other complications.

An implantable medical device embodying the present invention mitigates the risk of such complications, and others, by providing barbs that reduce in height and/or change angle when the implantable medical device transitions from an expanded to a constrained state.

During a recapture to remove and/or reposition, an expanded implantable medical device embodying the present invention may be drawn into a catheter and/or otherwise transitioned from an expanded to a constrained state. During the transition, the points of attachment of the secured ends of the fixation struts move away from the points of egress of the respective free barb ends. Thus, in the constrained state a greater distance separates a struts point of attachment and its point of egress. Accordingly, in some embodiments, as the implantable medical device transitions from an expanded to constrained state, the main body acts like a tether pulling on the secured ends of the fixation struts reducing the amount and/or changing the angle the barb free ends protrude from the main body. The resulting reduction in height and/or change in angle of the barb free ends may cause an easier and/or less traumatic detachment from the surrounding tissue. In combination or the alternative, the reduction in height and/or change in the angle the free barb ends protrude the main body may lessen damage inflicted on inner surfaces of the catheter utilized for recapture or otherwise reduce the likelihood of complications resulting from damage to the catheter.

These and other embodiments which characterize the invention are pointed out with particularity in the claims annexed hereto and forming a part hereof. However, for a better understanding of the invention, its advantages and objectives obtained by its use, reference can be made to the drawings which form a further part hereof and the accompanying descriptive matter, in which there are illustrated and described various embodiments of the invention.

### BRIEF DESCRIPTION OF THE DRAWINGS

A detailed description of the invention is hereafter described with specific reference being made to the drawings.
Figure 1A depicts an expanded state of an example of the present disclosure not being part of the invention.
Figure 1B depicts a constrained state of the example depicted in Figure 1A.
Figure 2A depicts an embodiment in which a hole extending through a rib of the main body serves as a point of egress.
Figure 2B depicts an example in which an eyelet serves as a point of egress.
Figure 2C depicts an example in which a space provided when at least two ribs intertwine serves as a point of egress.
Figure 2D depicts an embodiment in which an opening in a closed cell pattern serves as a point of egress.
Figure 2E depicts an example in which an intersection of two ribs may serves as a point of egress.
Figure 3 depicts an example in which at least two of the fixation struts are secured to main body at an axial location.
Figure 4A depicts the expanded state of an embodiment in which a proximal displacement of a distal nexus, to which the ends of fixation struts are secured, reduces the height and/or changes the angle the free barbs ends of the fixation struts project from the main body during transition from an expanded state to a constrained state.
Figure 4B depicts a constrained state of the embodiment depicted in Figure 4A.
Figure 4C depicts movement of the struts as the embodiment depicted in Figure 4A transitions to the constrained state depicted in Figure 4B.
Figure 5A depicts the expanded state of an embodiment in which a distal displacement of a hub connecting secured ends of the fixation struts to the main body reduces the height and/or changes the angle the free barbs ends of the fixation struts project from the main body during transition from an expanded state to a constrained state.
Figure 5B depicts a constrained state of the embodiment depicted in Figure 5A.
Figure 5C depicts a possible embodiment of a hub connecting secured ends of the fixation struts to the main body.

### DETAILED DESCRIPTION OF THE INVENTION

While this invention may be embodied in many different forms, there are described in detail herein specific embodiments of the invention and further examples which are not part of the invention. This description is an exemplification of the principles of the invention and is not intended to limit the invention to the particular embodiments illustrated.

For the purposes of this disclosure, like reference numerals in the figures shall refer to like features unless otherwise indicated.

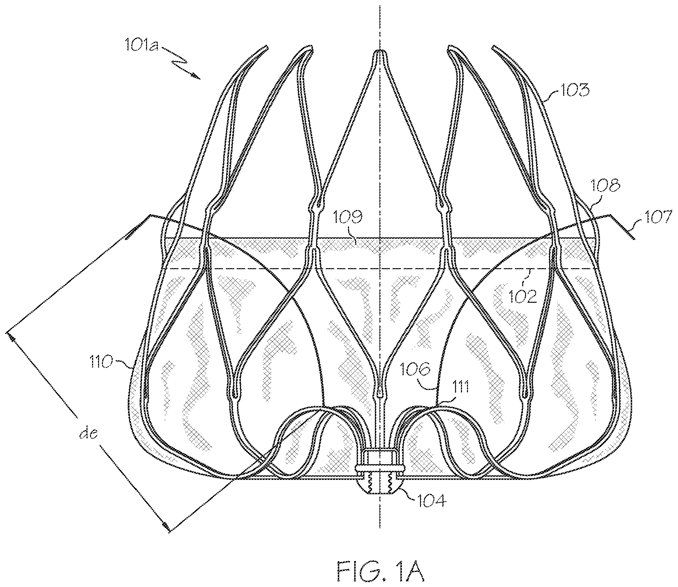

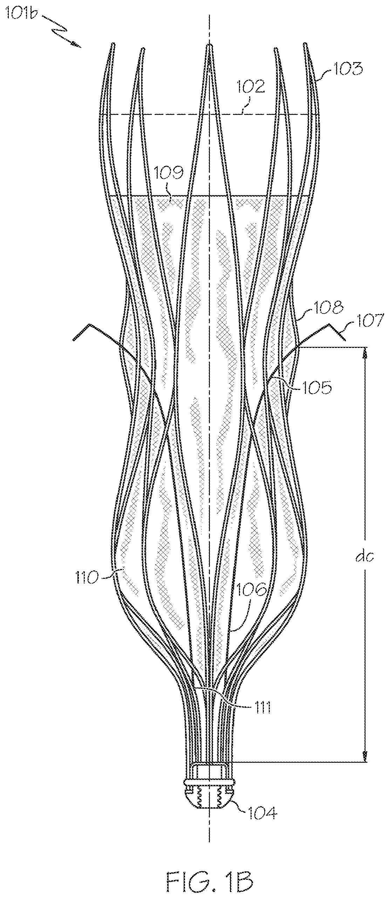

Figure 1 depicts an example of the present disclosure comprising a main body structure 101. As shown in Figures 1A and 1B, the example depicted has an expanded state 101a, Figure 1A, and a constrained state 101b, Figure 1B. The outer perimeter, shown by dashed line 102, of main body 101 is larger in the expanded stated than when in the constrained state. Main body 101 is formed from a plurality of ribs 103 extending radially outwards from nexus 104 and defining an interior 109. A plurality of fixation struts 105 are within main body 101. Each of the fixation struts 105 has a secured end 106 attached to the main body 101 at a point of attachment 111 and a free barb end 107 exiting the main body from a point of egress 108 and extending radially outwards past the outer perimeter. As shown in Figure 1A, fixation struts 105 extend through and across interior 109 of main body 101 in the expanded state.

Ribs 103 and/or fixation struts 105 may be formed from any suitable elastic material, for example, nitinol or spring steel. In the case of shape memory materials such as nitinol, the device may be provided with a memorized shape and then deformed to a reduced diameter shape. The device may restore itself to its memorized shape upon being heated to a transition temperature and/or having any restraints removed therefrom.

Depending on the specific examples and the requirements for the intended use, ribs 103 and/or fixation struts 105 may also be made from any other suitable biocompatible material including one or more polymers, one or more metals or combinations of polymer(s) and metal(s). Examples of suitable materials include biodegradable materials that are also biocompatible. In this context, the term "biodegradable" is used to denominate a material that undergoes breakdown or decomposition into harmless compounds as part of a normal biological process. Suitable biodegradable materials include polylactic acid, polyglycolic acid (PGA), collagen or other connective proteins or natural materials, polycaprolactone, hylauric acid, adhesive proteins, copolymers of these materials as well as composites and combinations thereof and combinations of other biodegradable polymers. Other polymers that may be used include polyester and polycarbonate copolymers. Examples of suitable metals include, but are not limited to, stainless steel, titanium, tantalum, platinum, tungsten, gold and/or alloys of any of the abovementioned metals. Examples of suitable alloys may include platinum-iridium alloys, cobalt-chromium alloys (e.g., Elgiloy and Phynox, MP35N), nickel-titanium alloys and nickel-titanium- platinum alloys.

In combination or the alternative, fixation members 105 may be formed from hypotubes.

An occlusion fabric 110 secured to the proximal end of main body 101 may aid in the prevention of blood flow and/or prevent the passage of large embolic material. Occlusion fabric 110 may be a permeable or impermeable. Fabric 110 may be made form a biocompatible material and/or may be made of a blood-permeable material having fluid conductive holes or channels extending across the membrane. These materials include, for example, ePFTE (e.g., Gore-Tex®), polyester (e.g., Dacron®), PTFE (e.g., Teflon®), silicone, urethane, metal fibers, and other biocompatible polymers. The size of the holes in the blood-permeable material may be chosen to be sufficiently small so that harmful-size emboli are filtered out from the blood flow between the appendage and the atrium. Suitable hole sizes may range, for example, from about 50 to about 400 microns in diameter. In embodiments, the filter membrane may be made of polyester (e.g., Dacron®) weave or knit having a nominal hole size of about 125 microns. The open area of the filter membrane (i.e., the hole density) may be selected or tailored to provide adequate flow conductivity for emboli-free blood to pass through the atrial appendage ostium. Further, portions of filter membrane may be coated or covered with an anticoagulant, such as heparin or another compound, or otherwise treated so that the treated portions acquire antithrombogenic properties to inhibit the formation of hole-clogging blood clots.

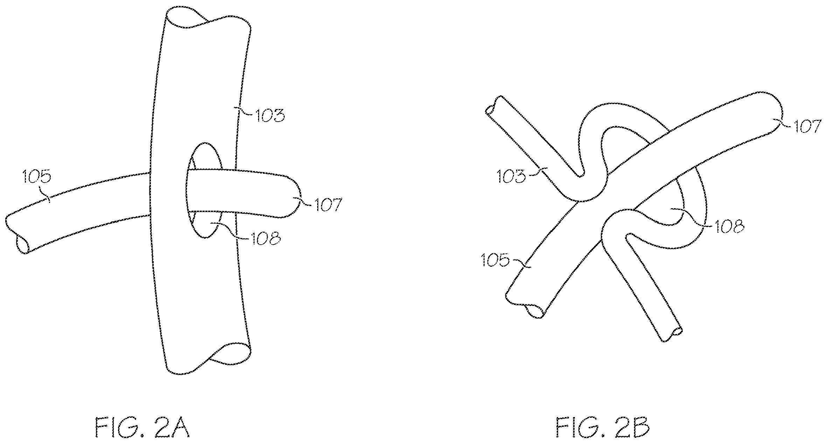

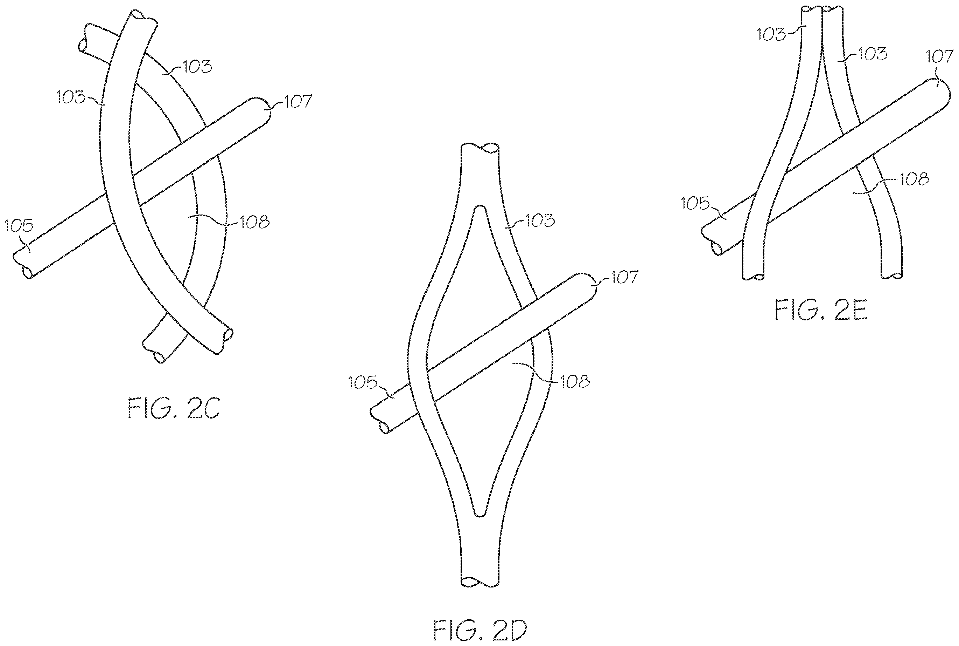

As shown in greater detail in Figure 2A, a point of egress 108 may be a passage or any opening extending through a rib 103 through which a free barb end 107 of at least one fixation strut 105 may pass. The passage shown in Figure 2A may be formed by cutting, drilling or otherwise providing a hole through rib 103. In combination or the alternative, point of egress 108 may be formed by bending a rib 103 to from an eyelet or similar structure, as shown in Figure 2B. Point of egress 108 may also be formed from space provided when at least two ribs 103 are braided, woven, latticed and/or otherwise intertwined, as shown in Figure 2C. Point of egress 108, in combination or the alternative, may also, as shown in Figure 2D, be an opening in a closed cell pattern. In other examples, as shown in Figure 2E, an intersection of two ribs may serves as point of egress 108.

As shown in Figure 1, the distance separating the point of attachment 111 of a fixation strut 105 and the point of egress 108 for the respective free barb 107 is greater in the constrained state, d_{c}, than the expanded state dₑ. Accordingly, as main body 101 transitions from the expanded state to the constrained state the secured ends 106 of each fixation strut 105 are displaced proximally with respect to the respective point of egress 108. The proximal displacement draws the free barb ends 107 inwards to main body 101 thereby reducing the amount each free bard end 107 protrudes from main body 101. During transition from the expanded state to the constrained, the main body 101 thus acts like a tether pulling on the secured ends 106 of fixation struts 105, reducing the height and/or changing the angle free barb ends 107 projects from main body 101. The resulting reduction and/or change in angle may cause an easier detachment of the depicted implantable medical device from the surrounding tissue when repositioning, removing and/or recapturing via a catheter.

The main body may act like a tether when the fixation members are secured at locations other than those depicted in Figure 1. For example, secured ends 106 of fixation struts 105 need not be attached to main body 101 at the same latitude. Likewise, points of egress 108 for free barb ends 107 of fixation struts 105 need not be positioned at the same latitude. Additionally, a secured end 106 of a fixation member 105 may be connected to the main body at an axial location.

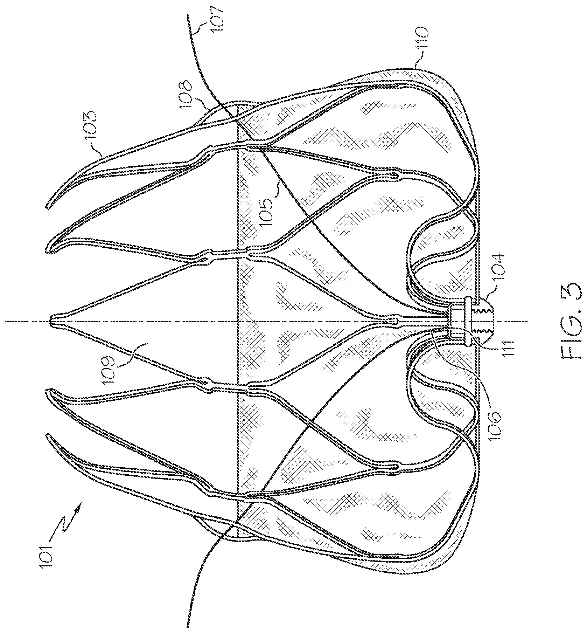

Figure 3 depicts an example in which at least two of the fixation struts 105 are secured to main body 101 at an axial location. In the example shown, the points of attachment 111 for secured ends 106 are located at nexus 104. Free barb ends 107, directed radially outwards from nexus 104, exit main body 101 at points of egress 108 and projecting radially outwards past outer perimeter 102. Accordingly, as shown in Figure 3, each fixation struts 105 extends through and across a portion of interior 109. When nexus 104 is displaced proximally, such as when the depicted implantable medical device is pulled into a catheter during recapture, nexus 104 pulls on secured ends 106 like a tether reducing the height and/or changing the angle free barb ends 107 project from main body 101. The resulting reduction in the amount and/or change in angle free barb ends 107 protrude from main body 101 may reduce fixation on the surrounding tissue permitting an easier and/or less traumatic detachment of the depicted implantable medical device from the surrounding tissue.

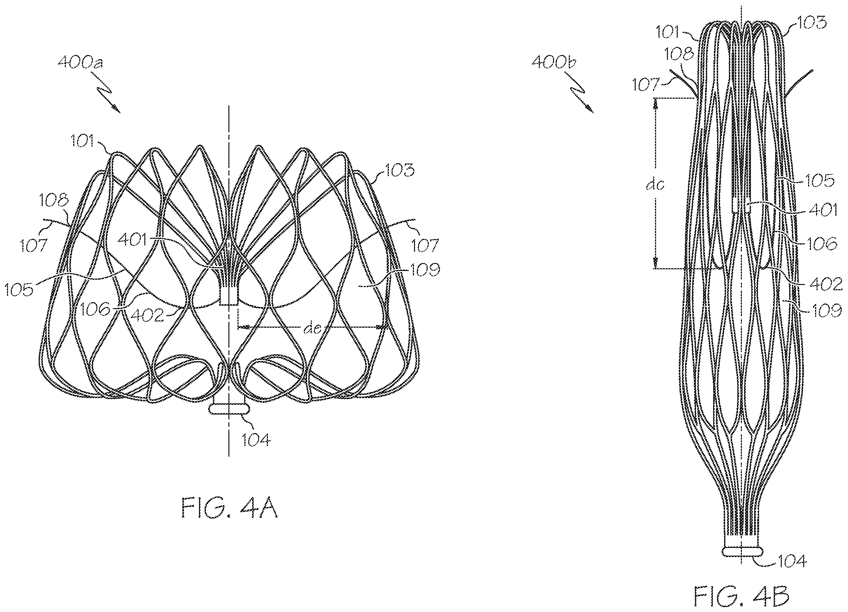

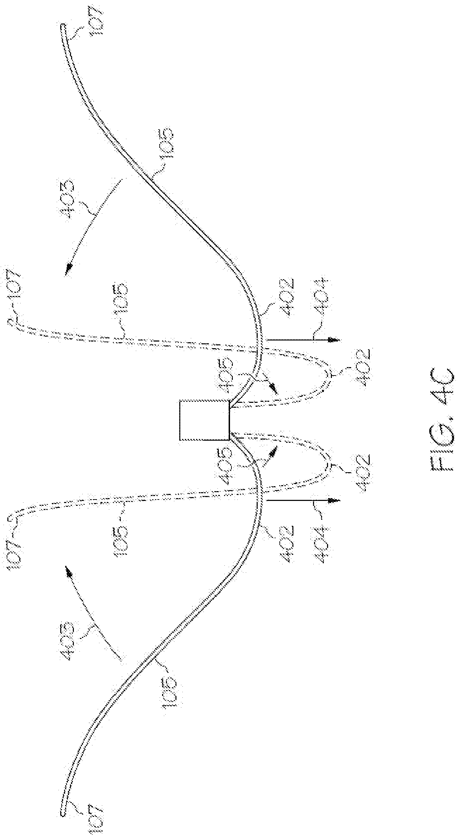

The points of attachment for the secured ends of the fixation struts, in combination or the alternative, may be located at a distal nexus as shown in Figure 4. The embodiment depicted in Figure 4 contains a main body 101 having a caged serpentine structure formed from a plurality of ribs 103 extending radially outwards from nexus 104 at the proximal end and combining at second nexus 401 at the distal end. Fixation struts 105 are formed from the distal terminations of ribs 103. As shown in Figure 4, at least some of ribs 103 contain a bend 402 directing free barb ends 107 to exit main body 101 from points of egress 108 and extend radially outwards past the outer perimeter 102. Accordingly, fixation struts 105 extend across and through a portion of interior 109 of main body 101 in the expanded state. As bends 402 connect fixation struts 105 to main body 101, bends 402 represent one possible embodiment of point of attachments in which fixation struts 105 are an integrated part of at least a portion of ribs 103.

As shown in Figures 4A and 4B, the embodiment depicted has an expanded state 400a, Figure 4A, and a constrained state 400b, Figure 4B. When the embodiment depicted in Figure 4 transitions from the expanded state 400a to the constrained state 400b, nexus 401 is displaced proximally with respect to points of egress 108 as main body 101 constricts, causing movement of the fixation struts as depicted in Figure 4C. The constriction of main body 101 pushes fixation struts 105 to a more vertical orientation, indicated by dashed lines, in the direction of arrows 403. Movement of fixation struts 105 along arrows 403 compresses bends 402 resulting in the proximal displacement of bends 402 in the direction of arrows 404. Compression of bends 402, in turn, induces rotation of their terminal ends about nexus 401 along the paths indicated by arrows 405.

As shown in Figures 4A and B, the transition of fixations struts 105 to a more vertical orientation in the constrained state increases the distance (d_{c} in the constrained state and dₑ in the expanded state) separating bend 402 of a fixation strut 105 and the point of egress 108 for the respective free barb 107. Accordingly, as main body 101 transitions from expanded state 400a to constrained state 400b the points of attachments, bends 402, for secured ends 106 of each fixation struts 105 are displaced a greater distance from the respective points of egress 108. The displacement draws free barb ends 107 inwards to main body 101, thereby reducing the amount and/or changing the angle each free barb end 107 protrudes from main body 101. The resulting reduction and/or change in angle may cause an easier and/or less traumatic detachment of the depicted implantable medical device from the surrounding tissue when repositioning, removing and/or recapturing via a catheter. In combination or the alternative, the reduction in height and/or change in the angle free barb ends 107 protrude from main body 101 may lessen damage inflicted on inner surfaces of the catheter utilized for recapture or otherwise reduce the likelihood of complications resulting from damage to the catheter.

In combination or the alternative, fixation struts may be made integral to the main body 101 and/or ribs 103. For example, the fixation struts and the ribs being collectively cut into body 101. In combination or the alternative, at least a portion of the plurality of fixation struts 105 may be attached to main body 101 via an intermediary structure.

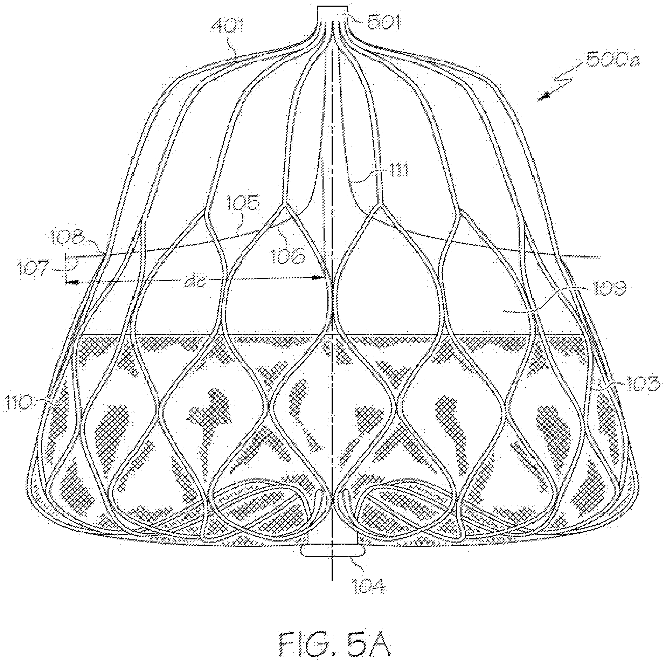

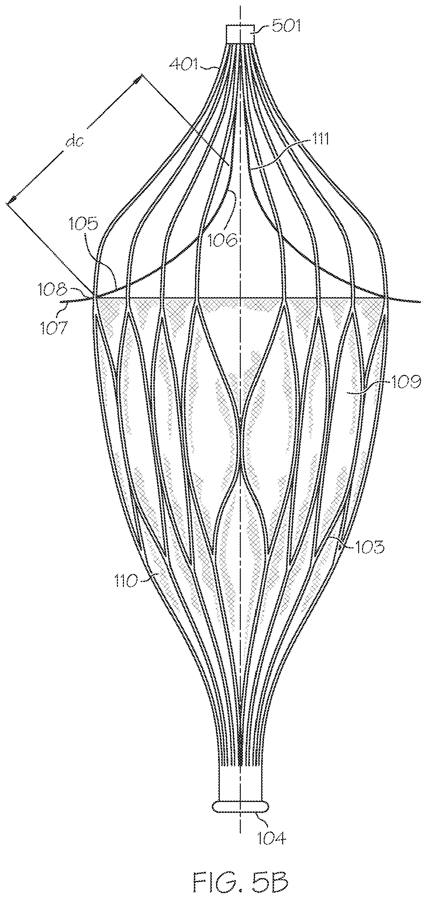

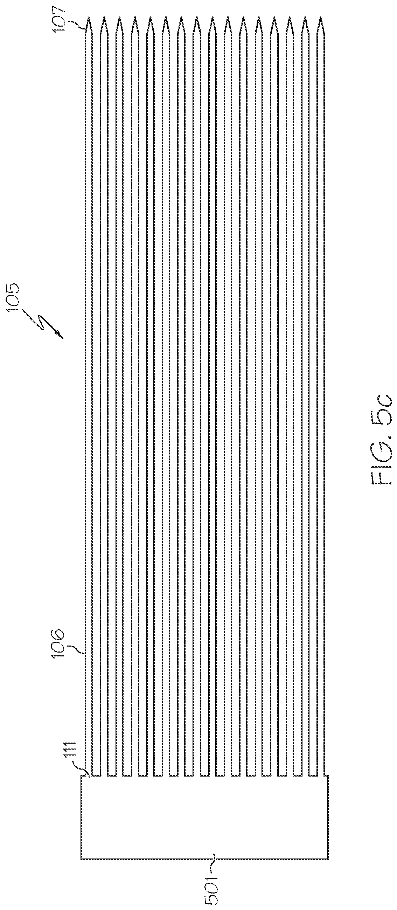

A proximal displacement of the points of attachment 111 of secured ends 106 is not necessary for reducing the amount and/or changing the angle free barb ends 107 protrude from main body 101 in a constrained state. In combination or the alternative, embodiments may utilize a distal displacement. Some embodiments of the present invention, additionally or in the alternative, may contain a hub connecting all or some of the fixation struts to the main body. In the embodiment shown in Figure 5 a distal displacement of hub 501, connecting secured ends 106 to nexus 401, reduces the amount and/or changes the angle free barb ends 107 protrude from main body 101. Hub 501 acts as a point of attachment connecting secured ends 106 of fixation members 105 to main body 101. As with the embodiment depicted in Figure 4, fixation struts 105 extend across and through a portion of interior 109 of main body 101 in the expanded state. Free barb ends 107 exiting main body 101 at points of egress 108 are directed radially outwards and project past the outer perimeter 102.

As shown in Figure 5C, hub 501 and fixation struts 105 may be integral components formed by laser cutting and/or other means readily recognizable to those skilled in the art.

As shown in Figures 5A and 5B, the embodiment depicted has an expanded state 500a, Figure 5A, and a constrained state 500b, Figure 5B. The distance separating the point of attachment 111 of a fixation strut 105 and the point of egress 108 for the respective free barb 107 is greater in the constrained state, d_{c}, than the expanded state dₑ. Accordingly, when nexus 401 is displaced distally, such as when the depicted implantable medical device is pulled into a catheter during recapture, nexus 401 via hub 501 pulls on secured ends 106 like a tether reducing the height and/or changing the angle of free barb ends 107. The resulting reduction in the amount and/or angle free barb ends 107 protrude from main body 101 may cause an easier and/or less traumatic detachment of the depicted implantable medical device from the surrounding tissue.

The above disclosure is intended to be illustrative and not exhaustive. This description will suggest many variations and alternatives to one of ordinary skill in this field of art.

Further, the particular features presented in the dependent claims can be combined with each other in other manners within the scope of the invention such that the invention should be recognized as also specifically directed to other embodiments having any other possible combination of the features of the dependent claims.

## Claims

1. A medical device comprising:
a proximal end;
a distal end;
a main body structure (101) having an outer perimeter (102) and an interior (109) defined by a plurality of ribs (103) extending radially outwards from a first nexus (104) at the proximal end and combining at a second nexus (401) at the distal end;
a fixation strut (105) extending through and across at least a portion of the interior of the main body (101) in an expanded state;
the fixation strut (105) being attached to the main body (101) at a point of attachment (111);
a barb (107) on the fixation strut (105), the barb (107) exiting the interior of the main body (101) at a point of egress (108) and projecting radially outwards a height from the main body (101) past the outer perimeter (102),
wherein a greater distance separates the point of attachment (111) and the point of egress (108) in a constrained state than the expanded state, reducing the height the barb end (107) projects from the main body (101);
**characterised in that** the medical device further comprises
a passage within at least one of the plurality of ribs (103), wherein the passage serves as the point of egress (108); and
an occlusion fabric (110) secured to the main body (101).

2. The medical device of claim 1, further comprising a hub connecting the fixation strut (105) with a second fixation strut (105).

3. The medical device of claim 2, wherein the first nexus (104) connects to the hub.

4. The medical device of claim 2, wherein the second nexus (401) connects to the hub.

5. The medical device of claim 1, wherein the main body (101) comprises a caged structure.

6. The medical device of claim 1, wherein the occlusion fabric (110) is blood-permeable.

7. The medical device of claim 1, wherein the plurality of ribs (103) form at least one serpentine structure capable of transitioning between the expanded state and the constrained state.

8. The medical device of claim 7, further comprising a cell within the serpentine structure of the main body (101), wherein the cell serves as the point of egress (108).

## Patentansprüche

1. Medizinische Vorrichtung, die aufweist:
ein proximales Ende;
ein distales Ende;
eine Hauptkörperstruktur (101) mit einem Außenumfang (102) und einem Inneren (109), die durch mehrere Rippen (103) definiert ist, die sich von einem ersten Nexus (104) an dem proximalen Ende radial auswärts erstrecken und sich an einem zweiten Nexus (401) an dem distalen Ende vereinen;
eine Fixierungsstrebe (105), die sich ein einem ausgedehnten Zustand durch und quer zu wenigstens einem Abschnitt des Inneren des Hauptkörpers (101) erstreckt;
wobei die Fixierungsstrebe (105) an einem Befestigungspunkt (111) an dem Hauptkörper (101) befestigt ist;
eine Spitze (107) an der Fixierungsstrebe (105), wobei die Spitze (107) das Innere des Hauptkörpers (101) an einem Austrittspunkt (108) verlässt und eine Höhe von dem Hauptkörper (101) an dem Außenumfang (102) vorbei radial nach außen vorsteht,
wobei in einem beschränkten Zustand ein größerer Abstand den Befestigungspunkt (111) und den Austrittspunkt (108) trennt als in dem ausgedehnten Zustand, was die Höhe, mit der das Spitzenende (107) von dem Hauptkörper (101) vorsteht, verringert;
**dadurch gekennzeichnet, dass** die medizinische Vorrichtung ferner aufweist:
einen Durchgang in wenigstens einer der mehreren Rippen (103), wobei der Durchgang als der Austrittspunkt (108) dient; und
ein Okklusionsgewebe (110), das an dem Hauptkörper (101) befestigt ist.

2. Medizinische Vorrichtung nach Anspruch 1, die ferner eine Nabe aufweist, welche die Fixierungsstrebe (105) mit einer zweiten Fixierungsstrebe (105) verbindet.

3. Medizinische Vorrichtung nach Anspruch 2, wobei der erste Nexus (104) mit der Nabe verbindet.

4. Medizinische Vorrichtung nach Anspruch 2, wobei der zweite Nexus (401) mit der Nabe verbindet.

5. Medizinische Vorrichtung nach Anspruch 1, wobei der Hauptkörper (101) eine Käfigstruktur aufweist.

6. Medizinische Vorrichtung nach Anspruch 1, wobei das Okklusionsgewebe (110) für Blut durchlässig ist.

7. Medizinische Vorrichtung nach Anspruch 1, wobei die mehreren Rippen (103) wenigstens eine gewundene Struktur bilden, die fähig ist, zwischen dem ausgedehnten Zustand und dem beschränkten Zustand zu wechseln.

8. Medizinische Vorrichtung nach Anspruch 7, die ferner eine Zelle innerhalb der gewundenen Struktur des Hauptkörpers (101) aufweist, wobei die Zelle als der Ausgangspunkt (108) dient.

## Revendications

1. Dispositif médical comprenant :
une extrémité proximale ;
une extrémité distale ;
une structure de corps principal (101) ayant un périmètre externe (102) et un intérieur (109) définie par une pluralité de nervures (103) s'étendant radialement vers l'extérieur à partir d'une première nexus (104) au niveau de l'extrémité proximale et se combinant au niveau d'une seconde nexus (401) au niveau de l'extrémité distale ;
une entretoise de fixation (105) s'étendant à travers et sur au moins une partie de l'intérieur du corps principal (101) dans un état expansé ;
l'entretoise de fixation (105) étant fixée sur le corps principal (101) à un point de fixation (111) ;
un ardillon (107) sur l'entretoise de fixation (105), l'ardillon (107) sortant de l'intérieur du corps principal (101) au niveau d'un point de sortie (108) et faisant saillie radialement vers l'extérieur sur une hauteur à partir du corps principal (101) au-delà du périmètre externe (102),
dans lequel une plus grande distance sépare le point de fixation (111) et le point de sortie (108) dans un état contraint que l'état expansé, réduisant la hauteur l'extrémité d'ardillon (107) fait saillie du corps principal (101) ;
**caractérisé en ce que** le dispositif médical comprend en outre :
un passage à l'intérieur d'au moins l'une de la pluralité de nervures (103), dans lequel le passage sert de point de sortie (108) ; et
un tissu d'occlusion (110) fixé sur le corps principal (101).

2. Dispositif médical selon la revendication 1, comprenant en outre un raccord raccordant l'entretoise de fixation (105) avec une seconde entretoise de fixation (105).

3. Dispositif médical selon la revendication 2, dans lequel la première nexus (104) se raccorde au raccord.

4. Dispositif médical selon la revendication 2, dans lequel la seconde nexus (401) se raccorde au raccord.

5. Dispositif médical selon la revendication 1, dans lequel le corps principal (101) comprend une structure à cage.

6. Dispositif médical selon la revendication 1, dans lequel le tissu d'occlusion (110) est perméable au sang.

7. Dispositif médical selon la revendication 1, dans lequel la pluralité de nervures (103) forme au moins une structure de serpentin pouvant effectuer une transition entre l'état expansé et l'état contraint.

8. Dispositif médical selon la revendication 7, comprenant en outre une cellule à l'intérieur de la structure de serpentin du corps principal (101), dans lequel la cellule sert de point de sortie (108).
